(19) Europäisches Patentamt
European Patent Office
Office européen des brevets

(11) EP 4 375 285 A1

(12) # EUROPEAN PATENT APPLICATION
published in accordance with Art. 153(4) EPC

(43) Date of publication:
**29.05.2024 Bulletin 2024/22**

(21) Application number: **21954827.8**

(22) Date of filing: **01.12.2021**

(51) International Patent Classification (IPC):
***C07D 477/20*** $^{(2006.01)}$ ***C07D 477/02*** $^{(2006.01)}$

(86) International application number:
**PCT/CN2021/134856**

(87) International publication number:
**WO 2023/024310 (02.03.2023 Gazette 2023/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.08.2021 CN 202110965003**

(71) Applicants:
- **Asymchem Laboratories (Tianjin) Co., Ltd.**
  **Tianjin 300457 (CN)**
- **Jilin Asymchem Pharmaceuticals Co., Ltd.**
  **Dunhua, Jilin 133700 (CN)**

(72) Inventors:
- **JAMES, Gage**
  **Morrisville North Carolina 27560 (US)**
- **MARK, McLaws**
  **Morrisville North Carolina 27560 (US)**
- **ZHANG, Wenchun**
  **Tianjin 300457 (CN)**
- **CHEN, Chaoyong**
  **Tianjin 300457 (CN)**
- **JING, Dingding**
  **Tianjin 300457 (CN)**
- **ZHANG, Baojie**
  **Tianjin 300457 (CN)**
- **JIANG, Yong**
  **Tianjin 300457 (CN)**

(74) Representative: **Finetti, Claudia**
**Bugnion S.p.A.**
**Viale Lancetti, 17**
**20158 Milano (IT)**

(54) **NEW CRYSTALLINE FORM OF ERTAPENEM SODIUM AND PREPARATION METHOD THEREFOR**

(57) The disclosure provides a new crystal form of ertapenem sodium and preparation method therefor. The new crystal form of ertapenem sodium has 27 principal characteristic peaks in an X-ray powder diffraction diagram. The crystal form of ertapenem sodium of the disclosure has a rod-like crystal habit, which has a large particle size, is not prone to aggregation and is easier to dry, and a product with high crystallinity and high purity may be obtained by simple drying treatment. Meanwhile, the crystal form of the disclosure has better stability, and the crystal form may remain unchanged to a maximum extent in subsequent washing and drying processes, thereby further improving the purity of the product.

EP 4 375 285 A1

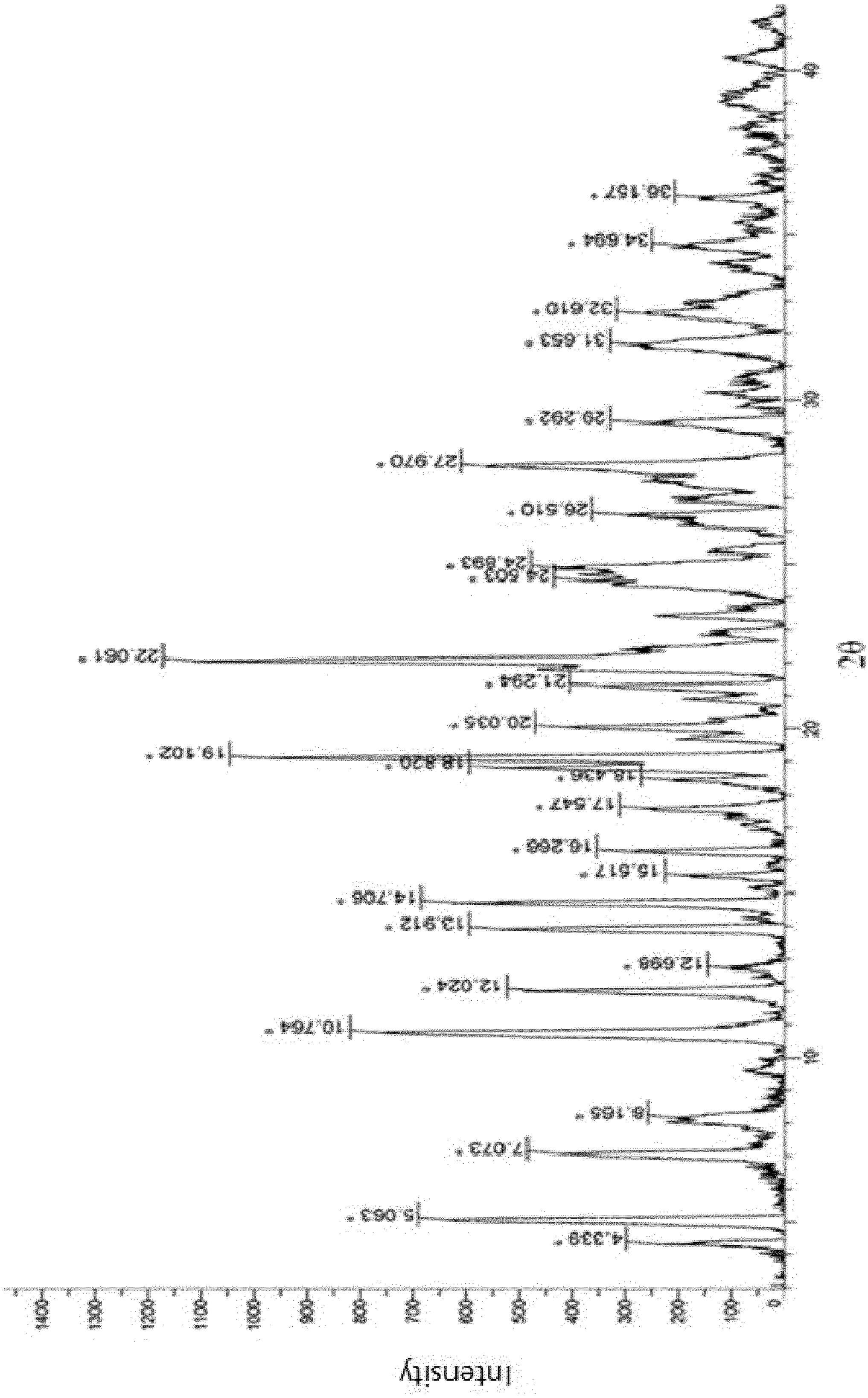
Intensity
2θ
4.339
5.063
7.073
8.165
10.764
12.024
12.698
13.912
14.706
15.517
16.266
17.547
18.436
18.820
19.102
20.035
21.294
22.061
24.503
24.893
26.510
27.970
29.292
31.653
32.610
34.694
36.157

Fig.1

## Description

### Technical Field

The disclosure relates to the field of organic synthesis, and specifically relates to a new crystal form of ertapenem sodium and preparation method therefor.

### Background

Ertapenem sodium has a structural formula of monosodium (4R,5S,6S)-3-[[[(3S,5S)-5-[(3-carboxyphenyl)amino]carbonyl]-3-pyrrolidinyl]thio]-6-[(1R)-1-hydroxy ethyl)-4-methyl-7-oxocarbonyl-1-azabicyclo[3.2.0]hept-2-ene-2-carboxylate. As first discovered by Merck & Co. (U.S.) and Astrazeneca, the ertapenem sodium has a wider range of activity than most of other β-lactam antibiotics and has higher drug resistance to β-lactamase, which are also served as main mechanisms of drug resistance in many bacteria. In clinical trials, compared with other carbapenems, the antibiotic has better pharmacokinetic characteristics, which allows for treatment with a single drug and a once-daily dose.

The crystal form is a key characteristic of all specific crystal products. Different crystal forms usually lead to different properties, such as stability, solubility and uniformity. The ertapenem sodium has poor stability under acidic, alkaline and thermal conditions, and needs to be dried and stored at low temperature during production.

At present, the ertapenem sodium has five different crystal forms. A crystal form A, a crystal form B and a crystal form C were first reported by Merck in patents WO03026572 and WO03027067. Detailed description of processes may refer to j.org.Chem (2005), 70, 7478-7487. According to the processes of Merck, the crystal form A was obtained by crystallization in a system containing methanol, water and n-propanol. The crystal form A has 21 characteristic peaks at a 2θ value of 4.8°, 6.7°, 10.5°, 11.7°, 13.6°, 14.4°, 16.0°, 17.2°, 18.4°, 19.7°, 20.8°, 21.6°, 22.1°, 23.1°, 24.1°, 26.1°, 26.6°, 27.0°, 27.4°, 28.6° and 31.1°. The crystal form A was stirred and washed in a system containing water and isopropanol (at a volume ratio of 15:85) to obtain the crystal form B. The crystal form B has 24 characteristic peaks at a 2θ value of 4.8°, 6.8°, 7.8°, 10.4°, 11.8°, 13.6°, 14.4°, 15.2°, 17.3°, 18.5°, 19.0°, 19.7°, 20.9°, 21.9°, 23.1°, 24.1°, 24.5°, 26.1°, 26.5°, 26.9°, 27.7°, 28.7°, 30.0°, 31.1° and 32.2°. When the crystal form B was stirred and washed in a methyl acetate (containing 2% of water) solution, the crystal form C was obtained. The crystal form C has 19 characteristic peaks at a 2θ value of 4.8°, 6.8°, 7.8°, 10.7°, 11.8°, 13.7°, 14.6°, 17.3°, 18.6°, 19.14°, 19.9°, 21.0°, 22.1°, 24.2°, 26.1°, 27.9°, 28.7°, 31.3° and 32.5°. A crystal form D was first reported in a patent WO2009150630. A crude product of ertapenem sodium was dissolved in pure water and methanol, and then crystallized with n-propanol. A resulting system was stirred at about 0°C to obtain a solid. The solid and acetone were stirred into a slurry to obtain a product of the crystal form D. The crystal form D has 11 characteristic peaks at a 2θ value of 4.44±0.2°, 5.26±0.2°, 7.44±0.2°, 8.12±0.2°, 10.98±0.2°, 12.74±0.2°, 19.28±0.2°, 22.93±0.2°, 23.51±0.2°, 25.07±0.2° and 30.15±0.2°, respectively. A crystal form E was first reported in a patent WO2013067878. A crude product of ertapenem sodium was dissolved in water (with a concentration of 50-90 mg/mL), and the pH of a resulting solution was adjusted to 5.4-5.5 with acetic acid. A solution containing methanol and n-propanol was added, and a resulting system was subjected to standing. Then, more methanol and n-propanol were added, and a resulting product was crystallized at low temperature to obtain the crystal form E. The crystal form E has 15 characteristic peaks at a 2θ value of 4.22±0.2°, 4.90±0.2°, 6.98±0.2°, 8.00±0.2°, 10.72±0.2°, 11.96±0.2°, 13.96±0.2°, 14.74±0.2°, 17.32±0.2°, 18.64±0.2°, 19.20±0.2°, 22.06±0.2°, 24.78±0.2°, 26.30±0.2° and 27.92±0.2°. A process for preparing amorphous ertapenem sodium was also disclosed in a patent CN1752090A. A filtrate was concentrated under vacuum, and then the pH was adjusted to 5.5. Then, methanol and n-propanol were added at a volume ratio of 1:1 to precipitate a product.

However, the processes for preparing the crystal form A, the crystal form B and the crystal form C involve a lot of extraction steps, so that the operation time is prolonged, and the processes have many impurities (such as dimers or ring-opening impurities) and lower product purity. In the process for preparing the crystal form D, due to particularly small particles, filtration is difficult, and accordingly, a product with high purity and a high yield cannot be obtained. In the process for preparing the crystal form E, due a small particle size and easy aggregation, the product is difficult to dry. In addition, since a large amount of solvents are used during preparation, a poor environmental protection effect is achieved. In the process for preparing the amorphous ertapenem sodium, the process has the main disadvantages of poor purity and stability of a product, which cannot be compared with other crystal forms.

In summary, crystal form products of ertapenem sodium in the prior art have low purity and a low yield. Therefore, it is necessary to provide a method for preparing a new crystal form product of ertapenem sodium, so as to achieve the advantages that the product has high purity and a high yield, and a preparation process is simple and easy to operate, which facilitates large-scale production in a factory easily.

## SUMMARY

The main purpose of the disclosure is to provide a new crystal form of ertapenem sodium and preparation method therefor, so as to solve the problems of low purity and low yield of crystal form products of ertapenem sodium in the prior art.

In order to achieve the above purpose, according to one aspect of the disclosure, a new crystal form of ertapenem sodium is provided, which has a structure shown in Formula I, and the new crystal form of ertapenem sodium has characteristic peaks at a diffraction angle (2θ) value of 4.3±0.2°, 5.1±0.2°, 7.1±0.2°, 8.2±0.2°, 10.8±0.2°, 12.0±0.2°, 12.7±0.2°, 13.9±0.2°, 14.7±0.2°, 15.5±0.2°, 16.3±0.2°, 17.5±0.2°, 18.4±0.2°, 18.8±0.2°, 19.1±0.2°, 20.0±0.2°, 21.3±0.2°, 22.1±0.2°, 24.5±0.2°, 24.9±0.2°, 26.5±0.2°, 28.0±0.2°, 29.3±0.2°, 31.7±0.2°, 32.6±0.2°, 34.7±0.2° and 36.2±0.2° in an X-ray powder diffraction diagram using a Cukα ray:

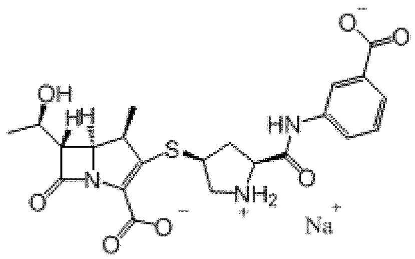

Formula I.

Further, the new crystal form of ertapenem sodium has characteristic peaks at the diffraction angle (2θ) value of 4.3°, 5.1°, 7.1°, 8.2°, 10.8°, 12.0°, 12.7°, 13.9°, 14.7°, 15.5°, 16.3°, 17.5°, 18.4°, 18.8°, 19.1°, 20.0°, 21.3°, 22.1°, 24.5°, 24.9°, 26.5°, 28.0°, 29.3°, 31.7°, 32.6°, 34.7° and 36.2° in the X-ray powder diffraction diagram using a Cukα ray.

Further, in the X-ray powder diffraction diagram of the new crystal form of ertapenem sodium, the characteristic peaks have an interplanar spacing and a relative height shown as follows:

| Serial number | 2θ (°) | Interplanar spacing d (Å) | Relative height (%) |
|---|---|---|---|
| 1 | 4.3 | 20.35 | 21 |
| 2 | 5.1 | 17.44 | 56 |
| 3 | 7.1 | 12.49 | 38 |
| 4 | 8.2 | 10.82 | 17 |
| 5 | 10.8 | 8.21 | 68 |
| 6 | 12.0 | 7.35 | 41 |
| 7 | 12.7 | 6.97 | 7 |
| 8 | 13.9 | 6.36 | 48 |
| 9 | 14.7 | 6.02 | 56 |
| 10 | 15.5 | 5.71 | 15 |
| 11 | 16.3 | 5.44 | 26 |
| 12 | 17.5 | 5.05 | 22 |
| 13 | 18.4 | 4.81 | 19 |
| 14 | 18.8 | 4.71 | 48 |
| 15 | 19.1 | 4.64 | 89 |
| 16 | 20.0 | 4.43 | 37 |

(continued)

| | | | |
|---|---|---|---|
| 17 | 21.3 | 4.17 | 31 |
| 18 | 22.1 | 4.03 | 100 |
| 19 | 24.5 | 3.63 | 33 |
| 20 | 24.9 | 3.57 | 37 |
| 21 | 26.5 | 3.36 | 27 |
| 22 | 28.0 | 3.19 | 49 |
| 23 | 29.3 | 3.05 | 24 |
| 24 | 31.7 | 2.82 | 24 |
| 25 | 32.6 | 2.74 | 23 |
| 26 | 34.7 | 2.58 | 17 |
| 27 | 36.2 | 2.48 | 13 |

In order to achieve the above purpose, according to one aspect of the disclosure, a preparation method for the new crystal form of ertapenem sodium is provided. The preparation method includes the following steps: adding a crude product of ertapenem sodium into an alkaline aqueous solution containing sodium ions to form a first system; adding methanol and n-propanol into the first system to form a second system; cooling the second system to -10°C to 15°C, and adding a mixed solution of an acid, methanol and n-propanol into the second system in 3-10 times to form a third system, where the volume of the mixed solution is 30-50% of the volume of the second system; cooling the third system to -30°C to -15°C, and heating the third system to -10°C to 15°C to form a fourth system; and then, sequentially subjecting the fourth system to filtration, washing treatment and drying treatment to obtain the new crystal form of ertapenem sodium; where the crude product of ertapenem sodium has a high performance liquid chromatography (HPLC) purity of 90-98%.

Further, in the mixed solution of an acid, methanol and n-propanol, the acid is acetic acid, formic acid, propionic acid, hydrochloric acid or hydrogen bromide; preferably, in the mixed solution of an acid, methanol and n-propanol, the volume ratio of the acid to the methanol to the n-propanol is 1:(10-20):(15-30); preferably, in the process of adding the mixed solution of an acid, methanol and n-propanol, the added amount is 10-35% of the volume of the mixed solution of an acid, methanol and n-propanol each time; and preferably, the time interval between two adjacent additions is 20-60 minutes.

Further, the crude product of ertapenem sodium has a concentration of 100-250 mg/mL in the first system.

Further, in the cooling process of the third system, the cooling is performed at a rate of 0.02-0.2°C/min; and preferably, in the heating process, the heating is performed at a rate of 0.02-0.2°C/min.

Further, the washing treatment includes sequentially performing a first washing process and a second washing process; preferably, a detergent used in the first washing process is a mixture of water, acetone and isopropanol; preferably, a detergent used in the second washing process is a mixture of acetone and water; more preferably, in the first washing process, the volume ratio of the water to the acetone to the isopropanol is 1:(1-7):(1-7); and more preferably, in the second washing process, the volume ratio of the acetone to the water is (90-99):(1-10).

Further, the alkaline aqueous solution containing sodium ions is a sodium bicarbonate aqueous solution, a sodium acetate aqueous solution or a sodium hydroxide aqueous solution; preferably, the alkaline aqueous solution containing sodium ions has a mass concentration of 20-50 mg/mL; and preferably, the molar ratio of alkali in the alkaline aqueous solution containing sodium ions to the crude product of ertapenem sodium is (0.9-1.5):1.

Further, before the third system is cooled, the method further includes a step of adding methanol and n-propanol into the third system; and preferably, the volume ratio of the methanol to the n-propanol is (4-7):(7-10).

The crystal form of the disclosure has a rod-like crystal habit, which has a large particle size and is not prone to aggregation. Therefore, the crystal form is easier to dry, and a product with high purity may be obtained by simple drying treatment. Meanwhile, the crystal form of the disclosure has better stability, and the crystal form may remain unchanged to a maximum extent in subsequent washing and drying processes, thereby further improving the purity of the product. In addition, since the crystal form of ertapenem sodium of the disclosure has a larger particle size and better stability, large-scale production in factory is easier, so that a product with high purity and a high yield can be obtained, where the purity of the product may be 99.2% or above, and the yield may be 93% or above.

## Brief Description of the Drawings

Drawings attached to the specification forming a part of the application are used to provide further understanding of the disclosure, and schematic embodiments of the disclosure and descriptions thereof are used to interpret the disclosure, rather than to form improper limitations of the disclosure. In the drawings:

Fig. 1 shows an X-ray powder diffraction diagram of a product in Example 1 of the disclosure;

Fig. 2 shows a scanning electron microscope photo (SEM, at a magnification of 500 times) of the product in Example 1 of the disclosure;

Fig. 3 shows a scanning electron microscope photo (SEM, at a magnification of 2,000 times) of the product in Example 1 of the disclosure;

Fig. 4 shows an X-ray powder diffraction diagram of a product in Example 2 of the disclosure;

Fig. 5 shows a scanning electron microscope photo (SEM, at a magnification of 500 times) of the product in Example 2 of the disclosure;

Fig. 6 shows a scanning electron microscope photo (SEM, at a magnification of 2,000 times) of the product in Example 2 of the disclosure;

Fig. 7 shows a coverage diagram of X-ray powder diffraction in Example 1 and Example 2 of the disclosure;

Fig. 8 shows a scanning electron microscope photo (SEM, at a magnification of 500 times) of a product in Comparative Example 5 of the disclosure; and

Fig. 9 shows a scanning electron microscope photo (SEM, at a magnification of 2,000 times) of the product in Comparative Example 5 of the present disclosure.

## Detailed Description of the Embodiments

Notably, the embodiments in the disclosure and features in the embodiments may be combined with each other without conflict. The disclosure is described in detail below with reference to the attached drawings and in conjunction with the embodiments.

As described in the background, crystal form products of ertapenem sodium in the prior art have low purity and a low yield. In order to solve the problems, the disclosure provides a new crystal form of ertapenem sodium, which has a structure shown in Formula I, and the new crystal form of ertapenem sodium has characteristic peaks at a diffraction angle (2$\theta$) value of 4.3±0.2°, 5.1±0.2°, 7.1±0.2°, 8.2±0.2°, 10.8±0.2°, 12.0±0.2°, 12.7±0.2°, 13.9±0.2°, 14.7±0.2°, 15.5±0.2°, 16.3±0.2°, 17.5±0.2°, 18.4±0.2°, 18.8±0.2°, 19.1±0.2°, 20.0±0.2°, 21.3±0.2°, 22.1±0.2°, 24.5±0.2°, 24.9±0.2°, 26.5±0.2°, 28.0±0.2°, 29.3±0.2°, 31.7±0.2°, 32.6±0.2°, 34.7±0.2° and 36.2±0.2° in an X-ray powder diffraction diagram using a Cuk$\alpha$ ray:

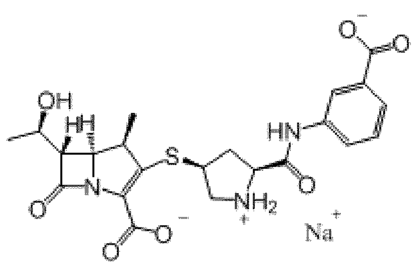

Formula I.

The crystal form of the disclosure has a rod-like crystal habit, which has a large particle size and is not prone to aggregation. Therefore, the crystal form is easier to dry, and a product with high purity may be obtained by simple

drying treatment. Meanwhile, the crystal form of the disclosure has better stability, and the crystal form may remain unchanged to a maximum extent in subsequent washing and drying processes, thereby further improving the purity of the product. In addition, since the crystal form of ertapenem sodium of the disclosure has a larger particle size and better stability, large-scale production in factory is easier, so that a product with high purity and a high yield can be obtained, where the purity of the product may be 99.2% or above, and the yield may be 93% or above.

In a preferred embodiment, the new crystal form of ertapenem sodium has characteristic peaks at a diffraction angle (2θ) value of 4.3°, 5.1°, 7.1°, 8.2°, 10.8°, 12.0°, 12.7°, 13.9°, 14.7°, 15.5°, 16.3°, 17.5°, 18.4°, 18.8°, 19.1°, 20.0°, 21.3°, 22.1°, 24.5°, 24.9°, 26.5°, 28.0°, 29.3°, 31.7°, 32.6°, 34.7° and 36.2° in the X-ray powder diffraction diagram using a Cukα ray. Further, in the X-ray powder diffraction diagram of the new crystal form of ertapenem sodium, the characteristic peaks have an interplanar spacing and a relative height shown as follows:

| Serial number | 2θ (°) | Interplanar spacing d (Å) | Relative height (%) |
|---|---|---|---|
| 1 | 4.3 | 20.35 | 21 |
| 2 | 5.1 | 17.44 | 56 |
| 3 | 7.1 | 12.49 | 38 |
| 4 | 8.2 | 10.82 | 17 |
| 5 | 10.8 | 8.21 | 68 |
| 6 | 12.0 | 7.35 | 41 |
| 7 | 12.7 | 6.97 | 7 |
| 8 | 13.9 | 6.36 | 48 |
| 9 | 14.7 | 6.02 | 56 |
| 10 | 15.5 | 5.71 | 15 |
| 11 | 16.3 | 5.44 | 26 |
| 12 | 17.5 | 5.05 | 22 |
| 13 | 18.4 | 4.81 | 19 |
| 14 | 18.8 | 4.71 | 48 |
| 15 | 19.1 | 4.64 | 89 |
| 16 | 20.0 | 4.43 | 37 |
| 17 | 21.3 | 4.17 | 31 |
| 18 | 22.1 | 4.03 | 100 |
| 19 | 24.5 | 3.63 | 33 |
| 20 | 24.9 | 3.57 | 37 |
| 21 | 26.5 | 3.36 | 27 |
| 22 | 28.0 | 3.19 | 49 |
| 23 | 29.3 | 3.05 | 24 |
| 24 | 31.7 | 2.82 | 24 |
| 25 | 32.6 | 2.74 | 23 |
| 26 | 34.7 | 2.58 | 17 |
| 27 | 36.2 | 2.48 | 13 |

The disclosure further provides a preparation method for the new crystal form of ertapenem sodium. The preparation method includes the following steps: adding a crude product of ertapenem sodium into an alkaline aqueous solution containing sodium ions to form a first system; adding methanol and n-propanol into the first system to form a second system; cooling the second system to -10°C to 15°C, and adding a mixed solution of an acid, methanol and n-propanol into the second system in 3-10 times to form a third system, where the volume of the mixed solution is 30-50%

of the volume of the second system; cooling the third system to -30°C to -15°C, and heating the third system to -10°C to 15°C to form a fourth system; and then, sequentially subjecting the fourth system to filtration, washing treatment and drying treatment to obtain the new crystal form of ertapenem sodium; where the crude product of ertapenem sodium has an HPLC purity of 90-98%.

In the disclosure, the new crystal form can be effectively controlled and obtained through coordination of the above operations. For the reasons described above, the crystal form prepared by the disclosure has a rod-like crystal habit, which has a large particle size and is not prone to aggregation. Therefore, the crystal form is easier to dry, and a product with high purity may be obtained by simple drying treatment. Meanwhile, the crystal form of the disclosure has better properties and stability, and the crystal form may remain unchanged to a maximum extent in subsequent washing and drying processes, so as to ensure the high purity of the product. The preparation method of the disclosure is simple and easy to operate and has better tolerance, which facilitates large-scale production in factory more easily.

In the disclosure, the crude product of ertapenem sodium is first pre-dissolved in the alkaline aqueous solution containing sodium ions to promote dissolution of most of ertapenem sodium. Then, the methanol and the n-propanol are added. On the one hand, the ertapenem sodium can be further completely dissolved. On the other hand, an environment can be provided for subsequent crystallization. Particularly, based on analysis of the crystal structure of the ertapenem sodium (a crystal compound of weakly bonded water), the ertapenem sodium is more sensitive to external perturbations due to multiple action forces between crystal water and molecules. In addition to factors such as solvent ratio and temperature, supersaturation is the most important factor affecting the crystal form. In the disclosure, by adding the mixed solution of an acid, methanol and n-propanol into the system in a batch mode, the system is always crystallized at a low supersaturation, and the low supersaturation can be continuously stabilized in the crystallization process. Thus, a good and stable environment can be created for assembly of unit cells of the crystal form. Accordingly, the new crystal form can be prepared by assembly, and improvement of the crystal crystallinity is facilitated. Furthermore, in the disclosure, through cooperation of the cooling and the heating in the crystallization process, impurities (such as ring-opening impurities, oxazinone, dimers and ring-opening methyl ester impurities) are dissolved in the heating process, and the product is precipitated in the cooling process, so that formation of the rod-like crystal habit and improvement of the crystallinity of the crystal form can be further optimized, and the crystal form has better stability. Specifically, the above impurities are derived from the following processes. After the ertapenem sodium is dissolved, the ring-opening impurities and the ring-opening methyl ester impurities are produced. The dimers are produced by dehydration of the one-molecule ertapenem and the one-molecule ring-opening impurities, and a total of 6-10 dimers are produced. The oxazinone is brought by the crude product of ertapenem sodium. In summary, by means of the preparation method, the new crystal form of ertapenem sodium with high purity and a high yield can be obtained in the disclosure, where the purity of the product may be 99.2% or above, and the yield may be 93% or above.

Notably, the crude product of ertapenem sodium, with a CAS No. 153773-82-1, may be selected as a raw material product in the disclosure.

Preferably, in the step of forming the fourth system, the cooling and the heating may be repeated for several times to further remove excess impurities and improve the purity of the product. More preferably, the cooling and the heating may be repeated for three times, so as to better balance energy consumption and high purity of the product.

In order to further effectively control the particle size of the crystal form, preferably, after the second system is cooled to -10°C to 5°C, the mixed solution of an acid, methanol and n-propanol is added.

In order to further effectively control the formation of the crystal form, preferably, in the preparation method, stirring may be performed at a rate of 100-300 r/min during formation of the first system, the second system and the third system. After a solid precipitate is observed in the third system, the stirring rate may be stabilized at 300 r/min.

In order to further improve the stability of the crystal form, preferably, crystal cultivation treatment is performed once after the mixed solution of an acid, methanol and n-propanol is added each time (the temperature of the system is remained unchanged, the stirring rate is remained unchanged at 300 r/min, and the time is 20-60 min). The crystal cultivation is performed for 3-10 times, which is consistent with the frequency of adding the mixed solution of an acid, methanol and n-propanol.

In order to further remove excess impurities and improve the purity of the product, more preferably, after the third system is cooled to -25°C to -20°C, the third system is heated to -10°C to 5°C.

In order to further effectively control the formation of the crystal form, preferably, in the mixed solution of an acid, methanol and n-propanol, the acid is acetic acid, formic acid, propionic acid, hydrochloric acid or hydrogen bromide; preferably, the molar ratio of the acid to the crude product of ertapenem sodium is (0.9-1.5):1; and preferably, in the mixed solution of acid, methanol and n-propanol, the volume ratio of the acid to the methanol to the n-propanol is 1 :(10-20):(15-30). Preferably, in the process of adding the mixed solution of an acid, methanol and n-propanol, the added amount is 10-35% of the volume of the mixed solution of an acid, methanol and n-propanol each time; and preferably, the time interval between two adjacent additions is 20-60 minutes.

In the cooling process of the third system, the cooling is performed at a rate of 0.02-0.2°C/min; and preferably, in the heating process, the heating is performed at a rate of 0.02-0.2°C/min.

In order to further improve the purity and yield of the product, preferably, the crude product of ertapenem sodium has a concentration of 100-250 mg/mL in the first system; and more preferably, the crude product of ertapenem sodium has a concentration of 100-150 mg/mL.

For the purpose of further improving the purity of the product, the washing treatment includes sequentially performing a first washing process and a second washing process; preferably, a detergent used in the first washing process is a mixture of water, acetone and isopropanol; preferably, a detergent used in the second washing process is a mixture of acetone and water; preferably, in the first washing process, the volume ratio of the water to the acetone to the isopropanol is 1:(1-7):(1-7); and preferably, in the second washing process, the volume ratio of the acetone to the water is (90-99):(1-10). More preferably, in the first washing process, the volume ratio of the water to the acetone to the isopropanol is 1 :(2-5):(4-7). More preferably, in the second washing process, the volume ratio of the acetone to the water is (95-98):(2-5). Preferably, the washing treatment is performed at a stirring rate of 50-500 r/min.

In order to further improve the solubility and stability of the crude raw material, preferably, the alkaline aqueous solution containing sodium ions is a sodium bicarbonate aqueous solution, a sodium acetate aqueous solution or a sodium hydroxide aqueous solution; and preferably, the alkaline aqueous solution containing sodium ions has a mass concentration of 20-50 mg/mL. Preferably, the molar ratio of an alkali in the alkaline aqueous solution containing sodium ions to the crude product of ertapenem sodium is (0.9-1.5):1. More preferably, in the step of forming the second system, the method further includes a step of adding methanol and n-propanol into the first system, where the volume ratio of the methanol to the n-propanol is (2-4):(2.5-4.5).

In order to further improve the crystallization efficiency and the product yield, before the third system is cooled, the method further includes a step of adding methanol and n-propanol into the third system, where the volume ratio of the methanol to the n-propanol is (4-7):(7-10).

Preferably, in the drying treatment process, the product is dried with wet nitrogen; preferably, the wet nitrogen has a relative humidity of 5-60%; and more preferably, the relative humidity is 10-30%. The product is dried with the wet nitrogen until a residual solvent meets ICH (International Conference on Harmonization of Technical Requirements for Registration of Pharmaceuticals for Human Use ) requirements and a moisture content meets specification requirements (KF: 15.5-19.0%).

The disclosure is further described in detail below in conjunction with specific embodiments, and the embodiments are not construed as limiting the scope of protection claimed by the disclosure.

Example 1

A preparation process is as follows.

A sodium bicarbonate solution was prepared (with 1,000 mL of pure water and 25.35 g of sodium bicarbonate), and 100 g of a crude product of ertapenem sodium (CAS: 153773-82-1; HPLC purity: 93%) was added into the solution to form a first system. The crude product of ertapenem sodium had a concentration of 100 mg/mL in the first system.

Methanol (280 mL) and n-propanol (350 mL) were added into the first system to form a second system.

At 0-5°C, a mixed solution of acetic acid, methanol and n-propanol (acetic acid: 18.15 g, methanol: 250 mL, n-propanol: 300 mL) was added into the second system in 8 times to form a third system. The added amount was 12.5% of the volume of the mixed solution of acetic acid, methanol and n-propanol each time; and the time interval between two adjacent additions was 30 minutes.

Methanol (1,260 mL) and n-propanol (2,265 mL) were added into the third system.

The third system was cooled to -25°C, then the third system was heated to 5°C, and the cooling and heating operations were repeated for two times to form a fourth system. The cooling was performed at a rate of 0.15°C/min, and the heating was performed at a rate of 0.15°C/min.

The fourth system was filtered for the first time, washed with isopropanol/acetone/pure water (700 mL/500 mU100 mL) at 0°C, filtered for the second time, washed with acetone/pure water (950 mL/50 mL) at 0°C, and filtered for the third time to obtain a solid product. Then, the product was dried with wet nitrogen (10-30%).

The product was detected by an X-ray diffractometer (Bruker D8 Focus). Then, the product was sampled and scanned at a 2θ value of 3° to 42° at a step size 0.02° (2θ), a tube voltage of 40 kV and a tube current of 40 mA.

An X-ray powder diffraction (XRPD) diagram of the product is shown in Fig. 1. A crystal form of the product has 27 principal characteristic peaks at a 2θ value of 4.3°, 5.1°, 7.1°, 8.2°, 10.8°, 12.0°, 12.7°, 13.9°, 14.7°, 15.5°, 16.3°, 17.5°, 18.4°, 18.8°, 19.1°, 20.0°, 21.3°, 22.1°, 24.5°, 24.9°, 26.5°, 28.0°, 29.3°, 31.7°, 32.6°, 34.7° and 36.2°. Specific data are shown in Table 1 below. According to determination of the product by HPLC, the ertapenem sodium has an HPLC purity of 99.4%.

Table 1

| Serial number | 2θ | Interplanar spacing d | Net intensity | Cross intensity | Relative height | Full width at half maximum | Width |
|---|---|---|---|---|---|---|---|
| 1 | 4.339° | 20.34689Å | 232 | 593 | 21.0% | 0.180 | 0.0898 |
| 2 | 5.063° | 17.44115Å | 623 | 905 | 56.4% | 0.191 | 0.0955 |
| 3 | 7.073° | 12.48692Å | 419 | 661 | 37.9% | 0.220 | 0.1098 |
| 4 | 8.165° | 10.81929Å | 191 | 421 | 17.3% | 0.371 | 0.1855 |
| 5 | 10.764° | 8.21219Å | 754 | 924 | 68.2% | 0.248 | 0.1241 |
| 6 | 12.024° | 7.35428Å | 458 | 613 | 41.4% | 0.172 | 0.0861 |
| 7 | 12.698° | 6.96545Å | 78.1 | 233 | 7.1% | 0.281 | 0.1403 |
| 8 | 13.912° | 6.36054Å | 528 | 694 | 47.8% | 0.186 | 0.0929 |
| 9 | 14.706° | 6.01878Å | 618 | 790 | 55.9% | 0.167 | 0.0835 |
| 10 | 15.517° | 5.70587Å | 160 | 332 | 14.5% | 0.130 | 0.0651 |
| 11 | 16.266° | 5.44483Å | 288 | 455 | 26.0% | 0.187 | 0.0937 |
| 12 | 17.547° | 5.05002Å | 245 | 419 | 22.2% | 0.193 | 0.0966 |
| 13 | 18.436° | 4.80840Å | 205 | 398 | 18.5% | 0.179 | 0.0897 |
| 14 | 18.820° | 4.71114Å | 528 | 731 | 47.7% | 0.211 | 0.1053 |
| 15 | 19.102° | 4.64226Å | 979 | 1189 | 88.5% | 0.271 | 0.1357 |
| 16 | 20.035° | 4.42825Å | 405 | 632 | 36.6% | 0.207 | 0.1036 |
| 17 | 21.294° | 4.16906Å | 339 | 576 | 30.6% | 0.209 | 0.1046 |
| 18 | 22.061° | 4.02588Å | 1106 | 1342 | 100.0% | 0.201 | 0.1003 |
| 19 | 24.503° | 3.62994Å | 369 | 623 | 33.4% | 0.160 | 0.0800 |
| 20 | 24.893° | 3.57386Å | 412 | 672 | 37.3% | 0.160 | 0.0800 |
| 21 | 26.510° | 3.35945Å | 298 | 566 | 26.9% | 0.425 | 0.2124 |
| 22 | 27.970° | 3.18735Å | 544 | 798 | 49.2% | 0.329 | 0.1644 |
| 23 | 29.292° | 3.04640Å | 264 | 518 | 23.9% | 0.247 | 0.1234 |
| 24 | 31.653° | 2.82438Å | 264 | 514 | 23.8% | 0.385 | 0.1923 |
| 25 | 32.610° | 2.74368Å | 251 | 484 | 22.7% | 0.333 | 0.1667 |
| 26 | 34.694° | 2.58348Å | 184 | 412 | 16.6% | 0.295 | 0.1474 |
| 27 | 36.157° | 2.48220Å | 141 | 374 | 12.8% | 0.209 | 0.4045 |

A solid sample was detected by SEM using a Phenom desktop scanning electron microscope (Phenom pure+). After sprayed in an ion sputtering device for 60 s, the solid was detected by a scanning electron microscope in an electron scanning mode. Sample results show no obvious aggregation, as shown in Fig. 2 and Fig. 3.

Data of the stability of the product at -18°C are shown in Table 2 below:

Table 2

| Time (month) | 0 | 1 | 2 | 3 | 4 | 5 | 6 | 7 | 8 | 9 | 10 | 11 | 12 |
|---|---|---|---|---|---|---|---|---|---|---|---|---|---|
| Purity (%) | 99.4 | 99.4 | 99.4 | 99.4 | 99.3 | 99.3 | 99.3 | 99.3 | 99.3 | 99.3 | 99.3 | 99.2 | 99.2 |
| Content (%) | 99.2 | 99.2 | 99.2 | 99.2 | 99.2 | 99.1 | 99.1 | 99.1 | 99.1 | 99.1 | 99.1 | 99.0 | 99.0 |

As can be seen from the data in Table 2, the crystal form product is very stable and is degraded at a slow rate.

Example 2

Different from Example 1, 20.28 g of sodium bicarbonate was used. A mixed solution of acetic acid, methanol and n-propanol (acetic acid: 14.52 g, methanol: 250 mL, n-propanol: 300 mL) was added into a second system in 3 times. A third system was cooled to -25°C, and then the third system was heated to 5°C to obtain a fourth system. Ertapenem sodium had a concentration of 100 mg/mL in a first system. The added amount was 33% of the volume of the mixed solution of acetic acid, methanol and n-propanol each time; and the time interval between two adjacent additions was 30 minutes. The cooling was performed at a rate of 0.12°C/min, and the heating was performed at a rate of 0.12°C/min. The cooling and the heating were only repeated for one time.

An X-ray powder diffraction (XRPD) diagram of the product is shown in Fig. 4. A crystal form of the product has 27 principal characteristic peaks at a 2θ value of 4.4°, 5.1°, 7.1°, 8.2°, 10.8°, 12.1°, 12.8°, 14.0°, 14.8°, 15.7°, 16.4°, 17.7°, 18.6°, 19.0°, 19.3°, 20.2°, 21.4°, 22.3°, 24.7°, 25.1°, 26.7°, 28.2°, 29.5°, 31.9°, 32.8°, 34.8° and 36.3°. Specific data are shown in Table 3 below. Fig. 7 shows a coverage diagram of X-ray powder diffraction in Example 1 and Example 2 of the disclosure. According to determination of the product by HPLC, the ertapenem sodium has an HPLC purity of 99.2%. A solid sample was detected by SEM using a Phenom desktop scanning electron microscope (Phenom pure+). After sprayed in an ion sputtering device for 60 s, the solid was detected by a scanning electron microscope in an electron scanning mode. Sample results show no obvious aggregation, as shown in Fig. 5 and Fig. 6.

Table 3

| Serial number | 2θ | Interplanar spacing d | Net intensity | Cross intensity | Relative height | Full width at half maximum | Width |
|---|---|---|---|---|---|---|---|
| 1 | 4.363 | 20.23566 | 355 | 663 | 31.9 | 0.165 | 0.0827 |
| 2 | 5.055 | 17.46818 | 1113 | 1401 | 100 | 0.14 | 0.0699 |
| 3 | 7.079 | 12.47618 | 692 | 945 | 62.1 | 0.154 | 0.0768 |
| 4 | 8.166 | 10.818 | 190 | 429 | 17 | 0.31 | 0.1552 |
| 5 | 10.834 | 8.15949 | 711 | 906 | 63.9 | 0.243 | 0.1215 |
| 6 | 12.099 | 7.30906 | 469 | 651 | 42.1 | 0.15 | 0.075 |
| 7 | 12.807 | 6.90674 | 78.8 | 256 | 7.1 | 0.225 | 0.1124 |
| 8 | 14.027 | 6.30864 | 767 | 956 | 68.9 | 0.155 | 0.0776 |
| 9 | 14.829 | 5.96916 | 835 | 1039 | 75 | 0.152 | 0.076 |
| 10 | 15.662 | 5.65332 | 241 | 456 | 21.6 | 0.161 | 0.0806 |
| 11 | 16.408 | 5.39796 | 272 | 492 | 24.4 | 0.155 | 0.0774 |
| 12 | 17.668 | 5.01568 | 200 | 429 | 18 | 0.273 | 0.1367 |
| 13 | 18.643 | 4.75554 | 156 | 399 | 14 | 0.218 | 0.109 |
| 14 | 18.978 | 4.67239 | 316 | 567 | 28.4 | 0.187 | 0.0937 |
| 15 | 19.326 | 4.58907 | 522 | 779 | 46.9 | 0.307 | 0.1533 |
| 16 | 20.211 | 4.39009 | 363 | 634 | 32.6 | 0.204 | 0.102 |
| 17 | 21.446 | 4.13987 | 293 | 576 | 26.3 | 0.212 | 0.1058 |
| 18 | 22.301 | 3.98318 | 561 | 844 | 50.4 | 0.304 | 0.1522 |
| 19 | 24.682 | 3.60401 | 300 | 586 | 26.9 | 0.16 | 0.08 |
| 20 | 25.055 | 3.55121 | 264 | 550 | 23.7 | 0.16 | 0.08 |
| 21 | 26.704 | 3.33555 | 159 | 438 | 14.3 | 0.589 | 0.2945 |
| 22 | 28.203 | 3.16153 | 304 | 568 | 27.3 | 0.314 | 0.1571 |
| 23 | 29.542 | 3.02121 | 135 | 391 | 12.1 | 0.381 | 0.1906 |
| 24 | 31.891 | 2.80386 | 154 | 411 | 13.9 | 0.418 | 0.2091 |
| 25 | 32.807 | 2.72761 | 145 | 395 | 13.1 | 0.16 | 0.08 |

(continued)

| Serial number | 2θ | Interplanar spacing d | Net intensity | Cross intensity | Relative height | Full width at half maximum | Width |
|---|---|---|---|---|---|---|---|
| 26 | 34.831 | 2.57362 | 108 | 354 | 9.7 | 0.442 | 0.2209 |
| 27 | 36.261 | 2.47532 | 77 | 323 | 6.9 | 0.251 | 0.1255 |

Data of the stability of the product at -18°C are shown in Table 4 below:

Table 4

| Time (month) | 0 | 1 | 2 | 3 | 4 | 5 | 6 | 7 | 8 | 9 | 10 | 11 | 12 |
|---|---|---|---|---|---|---|---|---|---|---|---|---|---|
| Purity (%) | 99.2 | 99.2 | 99.2 | 99.2 | 99.2 | 99.1 | 99.1 | 99.1 | 99.1 | 99.1 | 99.0 | 99.0 | 99.0 |
| Content (%) | 99.1 | 99.1 | 99.1 | 99.1 | 99.1 | 99.0 | 99.0 | 99.0 | 99.0 | 99.0 | 99.0 | 99.0 | 99.0 |

As can be seen from the data in Table 4, the crystal form product is very stable and is degraded at a slow rate.

Comparative Example 1

A crystal form A product of ertapenem sodium, a crystal form B product of ertapenem sodium and a crystal form C product of ertapenem sodium were prepared with reference to patents WO03026572 and WO03027067.

Comparative Example 2

A crystal form D product of ertapenem sodium was prepared with reference to a patent WO2009150630.

Comparative Example 3

A crystal form E product of ertapenem sodium was prepared with reference a patent WO2013067878.

Comparative Example 4

An amorphous product of ertapenem sodium was prepared with reference a patent CN1752090A.

Comparative Example 5

Ertapenem sodium with a purity of 98.4% was prepared with reference a patent CN102363617. Scanning electron microscope (SEM) photos of the ertapenem sodium are shown in Fig. 8 and Fig. 9. As shown in Fig. 8 and Fig. 9, the crystal form of ertapenem sodium has obvious aggregation, small particles and poor crystallinity. However, as shown in Fig. 2, Fig. 3, Fig. 5 and Fig. 6, the new crystal form products of the disclosure have a typical rod-like crystal habit, which have a large particle size and are not prone to aggregation.

Characterization of properties:

(One) 100 g (±5%) of the crystal form product in Example 1, the crystal form A product, the crystal form B product, the crystal form C product, the crystal form D product, the crystal form E product and the amorphous product of ertapenem sodium were separately taken and subjected to press filtration until no filtrate dropped, followed by drying until a solvent residue satisfied ICH standards. Corresponding filtration time and drying time are shown in Table 5.

Table 5

| | Filtration time | Drying time |
|---|---|---|
| Crystal form A | 63minutes | 35h |
| Crystal form B | -- | 23h |
| Crystal form C | -- | 11h |

(continued)

| | Filtration time | Drying time |
|---|---|---|
| Crystal form D | 110minutes | 15h |
| Crystal form E | 85minutes | 17h |
| Amorphous | 117minutes | 25h |
| Example 1 | 25minutes | 9h |
| Notes:<br>1. As the crystal form B was obtained by stirring and washing the crystal form A and the crystal form C was obtained by stirring and washing the crystal form B, only the filtration time of the crystal form A was counted.<br>2. The press filtration was performed by a same Hastelloy filter dryer (800 mL Φ=110 mm), it was ensured that the press filtration was performed at a vacuum degree (p) of equal to or less than -0.095 MPa, and meanwhile, an upper port of the filter dryer was protected with nitrogen.<br>3. The drying was performed at a temperature of 5-15°C, a nitrogen flow rate of 1-2 $m^3/h$ and a nitrogen humidity of 20-40%. | | |

As can be seen from the data in Table 5, the new crystal form product prepared by the disclosure has a rod-like crystal habit, which has a large particle size and is not prone to aggregation, so that the filtration efficiency and the drying efficiency are obviously improved, the production efficiency can be effectively improved in large-scale production, and meanwhile, the degradation risk of the product can be reduced.

(Two) A fluidity related test was carried out on the crystal form A product of ertapenem sodium, the crystal form B product, the crystal form C product, the crystal form D product, the crystal form E product and the amorphous product of ertapenem sodium. Test results are shown in Table 6.

Table 6

| | Tap density | Bulk density | Carr index | Hausner ratio | Angle of repose |
|---|---|---|---|---|---|
| Crystal form A | 0.82g/$cm^3$ | 0.64 g/$cm^3$ | 22% | 1.28 | 43° |
| Crystal form B | 0.79 g/$cm^3$ | 0.63 g/$cm^3$ | 20% | 1.25 | 41° |
| Crystal form C | 0.78 g/$cm^3$ | 0.63 g/$cm^3$ | 19% | 1.24 | 43° |
| Crystal form D | 0.81 g/$cm^3$ | 0.64 g/$cm^3$ | 21% | 1.27 | 47° |
| Crystal form E | 0.83 g/$cm^3$ | 0.66 g/$cm^3$ | 20% | 1.26 | 46° |
| Amorphous | 0.79 g/$cm^3$ | 0.63 g/$cm^3$ | 20% | 1.25 | 47° |
| Crystal form F | 0.71 g/$cm^3$ | 0.64 g/$cm^3$ | 10% | 1.11 | 23° |

A relationship between the Carr Index, the Hausner ratio and fluidity evaluation is shown in Table 7 below.

$$\text{Carr index} = (\text{tap density} - \text{bulk density}) / \text{tap density} \times 100\%.$$

Hausner ratio=tap density/bulk density.

Table 7

| Carr index | Hausner ratio | Fluidity evaluation |
|---|---|---|
| ≤10% | 1.00-1.11 | Extremely good |
| 11-15% | 1.12-1.18 | Good |
| 16-20% | 1.19-1.25 | Less good |
| 21-25% | 1.26-1.34 | Acceptable |
| 26-31% | 1.35-1.45 | Poor |

(continued)

| Carr index | Hausner ratio | Fluidity evaluation |
|---|---|---|
| 32-37% | 1.46-1.59 | Extremely poor |
| ≥38% | ≥1.60 | Highly extremely poor |

As can be seen from the data in Table 6 and Table 7, it can be judged that the new crystal form product of the disclosure has better fluidity when compared with the existing crystal form A, the crystal form B, the crystal form C, the crystal form D, the crystal form E and the amorphous product based on the Carr index, the Hausner ratio and the angle of repose. At present, the ertapenem sodium is mainly used in preparations in the form of a freeze-dried powder, and due to the better fluidity, infinite possibilities are provided for development and use of other potential dosage forms.

The above descriptions are only preferred embodiments of the disclosure and are not intended to limit the disclosure, and various alterations and changes of the disclosure can be made by persons skilled in the art. Any modifications, equivalent substitutions, improvements and the like made within the spirit and principles of the disclosure shall be included in the scope of protection of the disclosure.

## Claims

1. A new crystal form of ertapenem sodium, having a structure shown in Formula I, wherein the new crystal form of ertapenem sodium has characteristic peaks at a diffraction angle (2θ) value of 4.3±0.2°, 5.1±0.2°, 7.1±0.2°, 8.2±0.2°, 10.8±0.2°, 12.0±0.2°, 12.7±0.2°, 13.9±0.2°, 14.7±0.2°, 15.5±0.2°, 16.3±0.2°, 17.5±0.2°, 18.4±0.2°, 18.8±0.2°, 19.1±0.2°, 20.0±0.2°, 21.3±0.2°, 22.1±0.2°, 24.5±0.2°, 24.9±0.2°, 26.5±0.2°, 28.0±0.2°, 29.3±0.2°, 31.7±0.2°, 32.6±0.2°, 34.7±0.2° and 36.2±0.2° in an X-ray powder diffraction diagram using a Cukα ray:

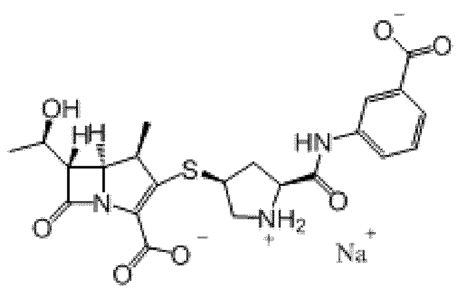

Formula I.

2. The new crystal form of ertapenem sodium according to claim 1, wherein the new crystal form of ertapenem sodium has characteristic peaks at the diffraction angle (2θ) value of 4.3°, 5.1°, 7.1°, 82°, 10.8°, 12.0°, 12.7°, 13.9°, 14.7°, 15.5°, 16.3°, 17.5°, 18.4°, 18.8°, 19.1°, 20.0°, 21.3°, 22.1°, 24.5°, 24.9°, 26.5°, 28.0°, 29.3°, 31.7°, 32.6°, 34.7° and 36.2° in the X-ray powder diffraction diagram using a Cukα ray.

3. The new crystal form of ertapenem sodium according to claim 1 or 2, wherein in the X-ray powder diffraction diagram of the new crystal form of ertapenem sodium, the characteristic peaks have an interplanar spacing and a relative height shown as follows:

| Serial number | 2θ (°) | Interplanar spacing d (Å) | Relative height (%) |
|---|---|---|---|
| 1 | 4.3 | 20.35 | 21 |
| 2 | 5.1 | 17.44 | 56 |
| 3 | 7.1 | 12.49 | 38 |
| 4 | 8.2 | 10.82 | 17 |

(continued)

| Serial number | 2θ (°) | Interplanar spacing d (Å) | Relative height (%) |
|---|---|---|---|
| 5 | 10.8 | 8.21 | 68 |
| 6 | 12.0 | 7.35 | 41 |
| 7 | 12.7 | 6.97 | 7 |
| 8 | 13.9 | 6.36 | 48 |
| 9 | 14.7 | 6.02 | 56 |
| 10 | 15.5 | 5.71 | 15 |
| 11 | 16.3 | 5.44 | 26 |
| 12 | 17.5 | 5.05 | 22 |
| 13 | 18.4 | 4.81 | 19 |
| 14 | 18.8 | 4.71 | 48 |
| 15 | 19.1 | 4.64 | 89 |
| 16 | 20.0 | 4.43 | 37 |
| 17 | 21.3 | 4.17 | 31 |
| 18 | 22.1 | 4.03 | 100 |
| 19 | 24.5 | 3.63 | 33 |
| 20 | 24.9 | 3.57 | 37 |
| 21 | 26.5 | 3.36 | 27 |
| 22 | 28.0 | 3.19 | 49 |
| 23 | 29.3 | 3.05 | 24 |
| 24 | 31.7 | 2.82 | 24 |
| 25 | 32.6 | 2.74 | 23 |
| 26 | 34.7 | 2.58 | 17 |
| 27 | 36.2 | 2.48 | 13 |

**4.** A preparation method for the new crystal form of ertapenem sodium according to any one of claims 1 to 3, wherein the preparation method comprises the following steps:

adding a crude product of ertapenem sodium into an alkaline aqueous solution containing sodium ions to form a first system;
adding methanol and n-propanol into the first system to form a second system;
cooling the second system to -10°C to 15°C, and adding a mixed solution of acid, methanol and n-propanol into the second system in 3-10 times to form a third system, wherein the volume of the mixed solution of acid, methanol and n-propanol is 30-50% of the volume of the second system;
cooling the third system to -30°C to -15°C, and heating the third system to -10°C to 15°C to form a fourth system;
and then, sequentially subjecting the fourth system to filtration, washing and drying treatment to obtain the new crystal form of ertapenem sodium;
wherein the crude product of ertapenem sodium has a high performance liquid chromatography (HPLC) purity of 90-98%.

**5.** The preparation method according to claim 4, wherein in the mixed solution of acid, methanol and n-propanol, the acid is acetic acid, formic acid, propionic acid, hydrochloric acid or hydrogen bromide.

**6.** The preparation method according to claim 5, wherein in the mixed solution of acid, methanol and n-propanol, the volume ratio of the acid to the methanol to the n-propanol is 1 :(10-20):(15-30).

7. The preparation method according to claim 5, wherein in the process of adding the mixed solution of acid, methanol and n-propanol, the added amount is 10-35% of the volume of the mixed solution of acid, methanol and n-propanol each time; and the time interval between two adjacent additions is 20-60 minutes.

8. The preparation method according to claim 4, wherein the crude product of ertapenem sodium has a concentration of 100-250 mg/mL in the first system.

9. The preparation method according to claim 4, wherein in the cooling process of the third system, the cooling is performed at a rate of 0.02-0.2°C/min.

10. The preparation method according to claim 4, wherein the washing treatment comprises sequentially performing a first washing process and a second washing process.

11. The preparation method according to claim 4, wherein the alkaline aqueous solution containing sodium ions is a sodium bicarbonate aqueous solution, a sodium acetate aqueous solution or a sodium hydroxide aqueous solution.

12. The preparation method according to claim 4, wherein before the third system is cooled, the method further comprises a step of adding methanol and n-propanol into the third system.

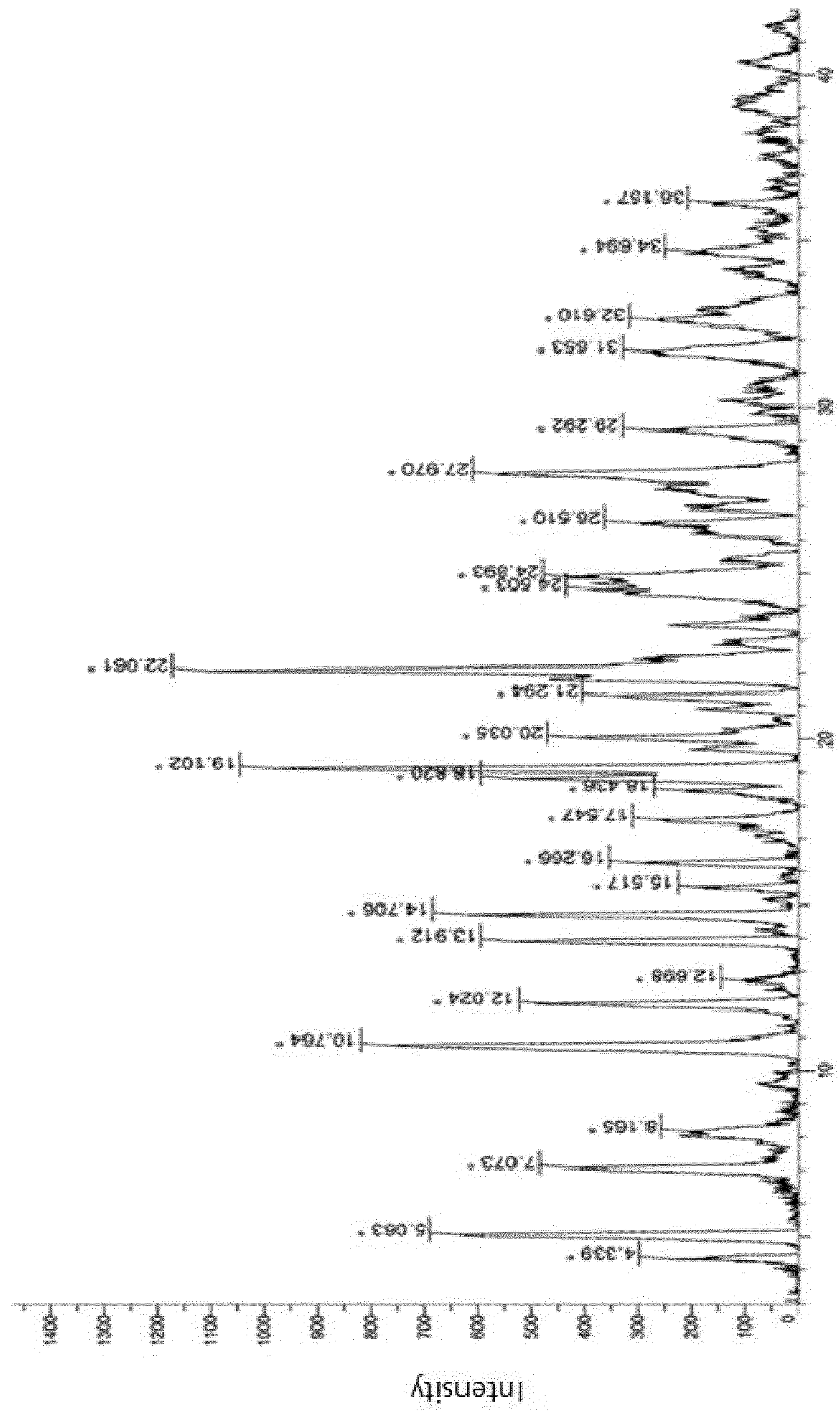
Intensity
2θ
4.339
5.063
7.073
8.165
10.764
12.024
12.698
13.912
14.706
15.517
16.266
17.547
18.436
18.820
19.102
20.035
21.204
22.061
24.503
24.893
26.510
27.970
29.292
31.653
32.610
34.694
36.157

Fig.1

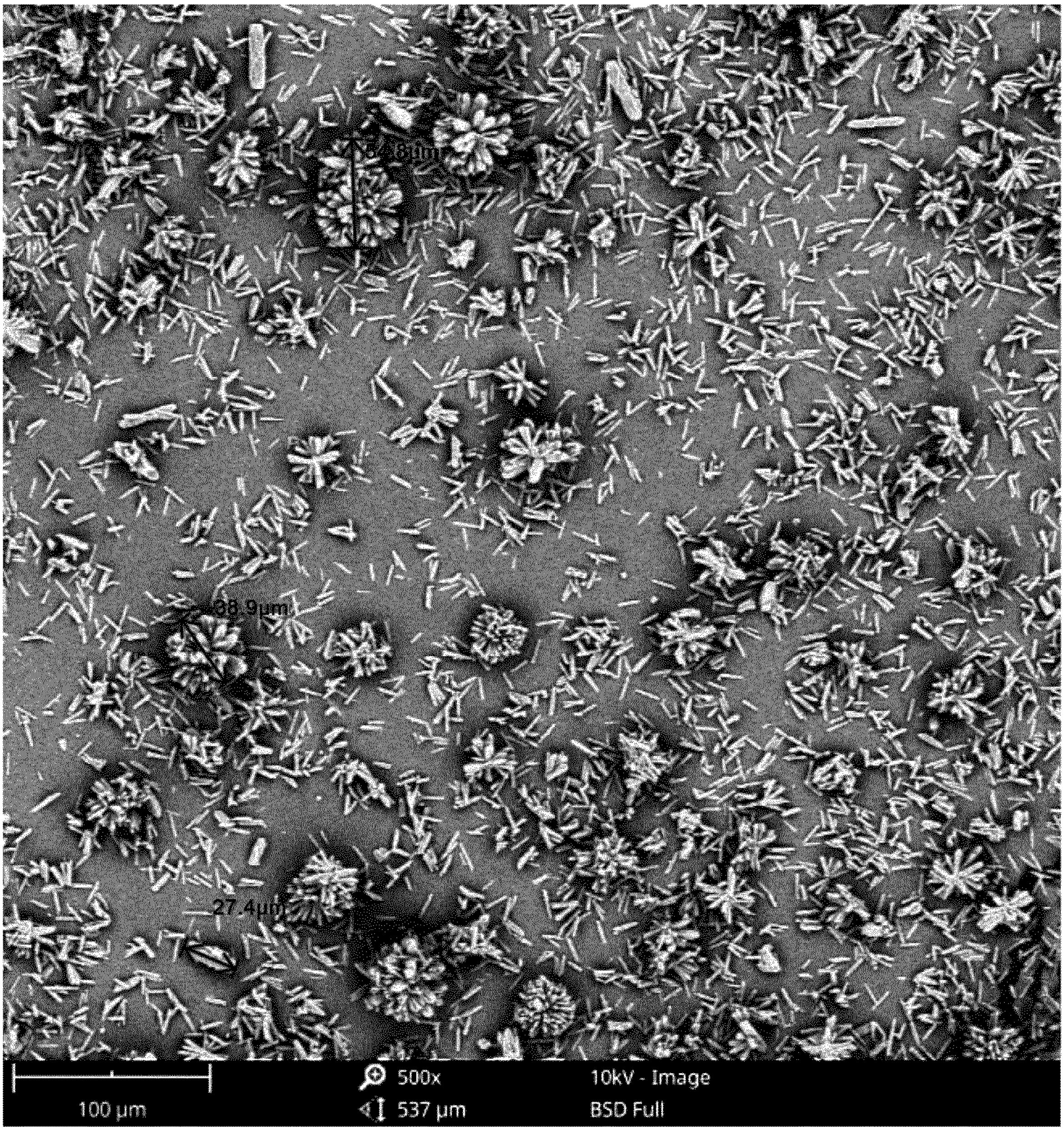


Fig.2

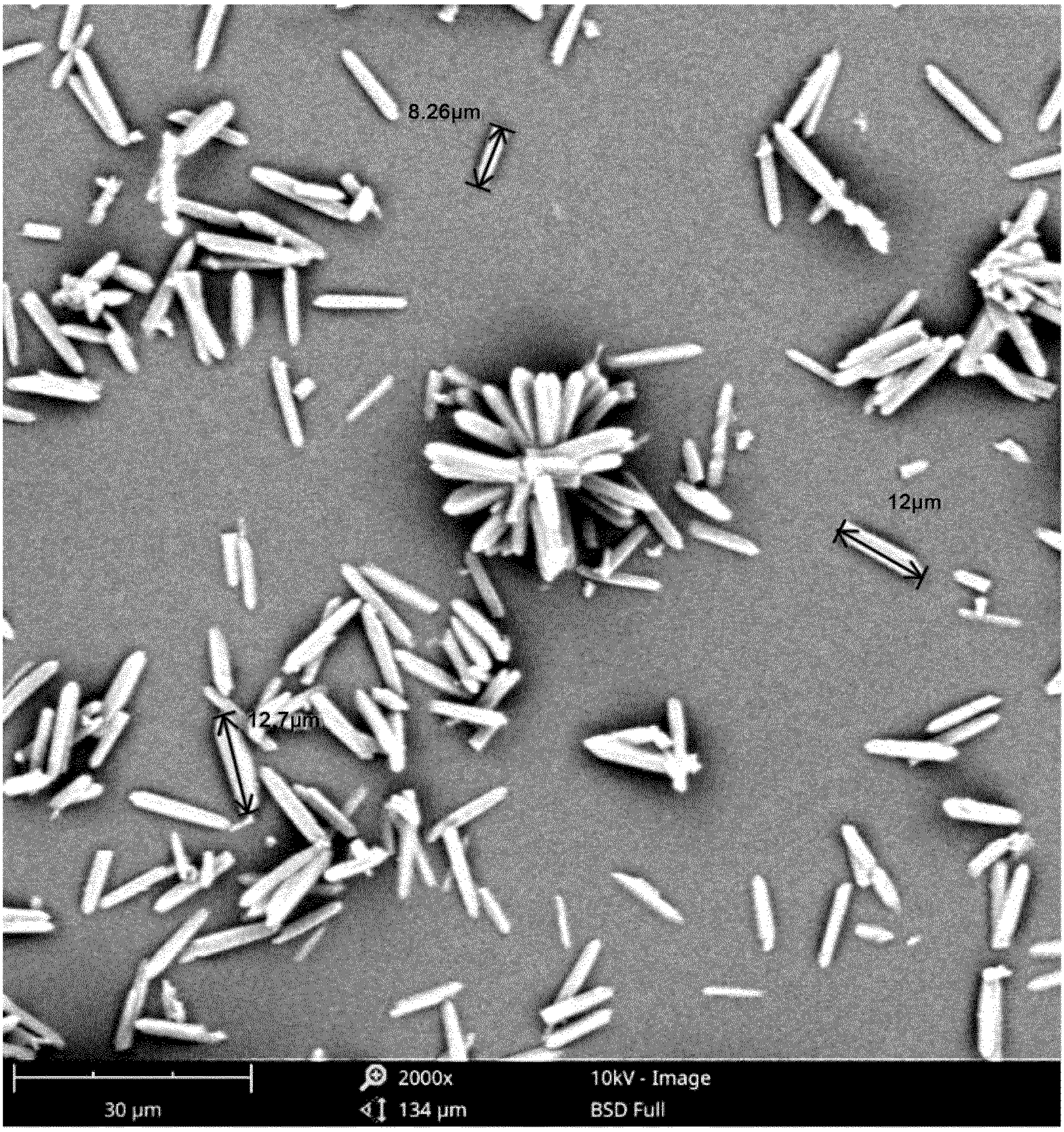


Fig.3

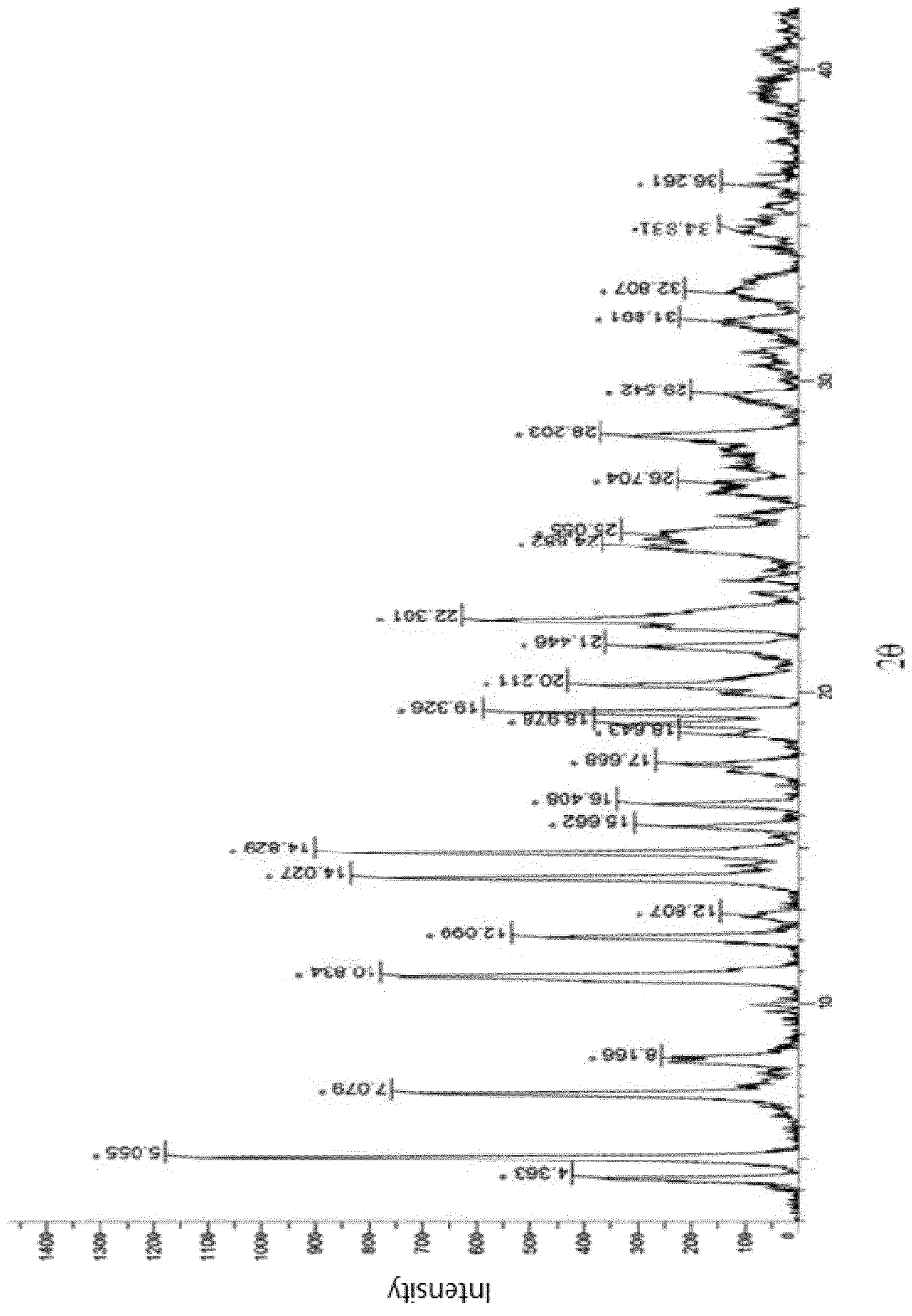

Intensity
2θ
4.363
5.055
7.079
8.166
10.834
12.099
12.807
14.027
14.829
15.662
16.408
17.668
18.643
18.928
19.326
20.211
21.446
22.301
24.882
25.055
26.704
28.203
29.542
31.801
32.807
34.831
36.261


Fig.4

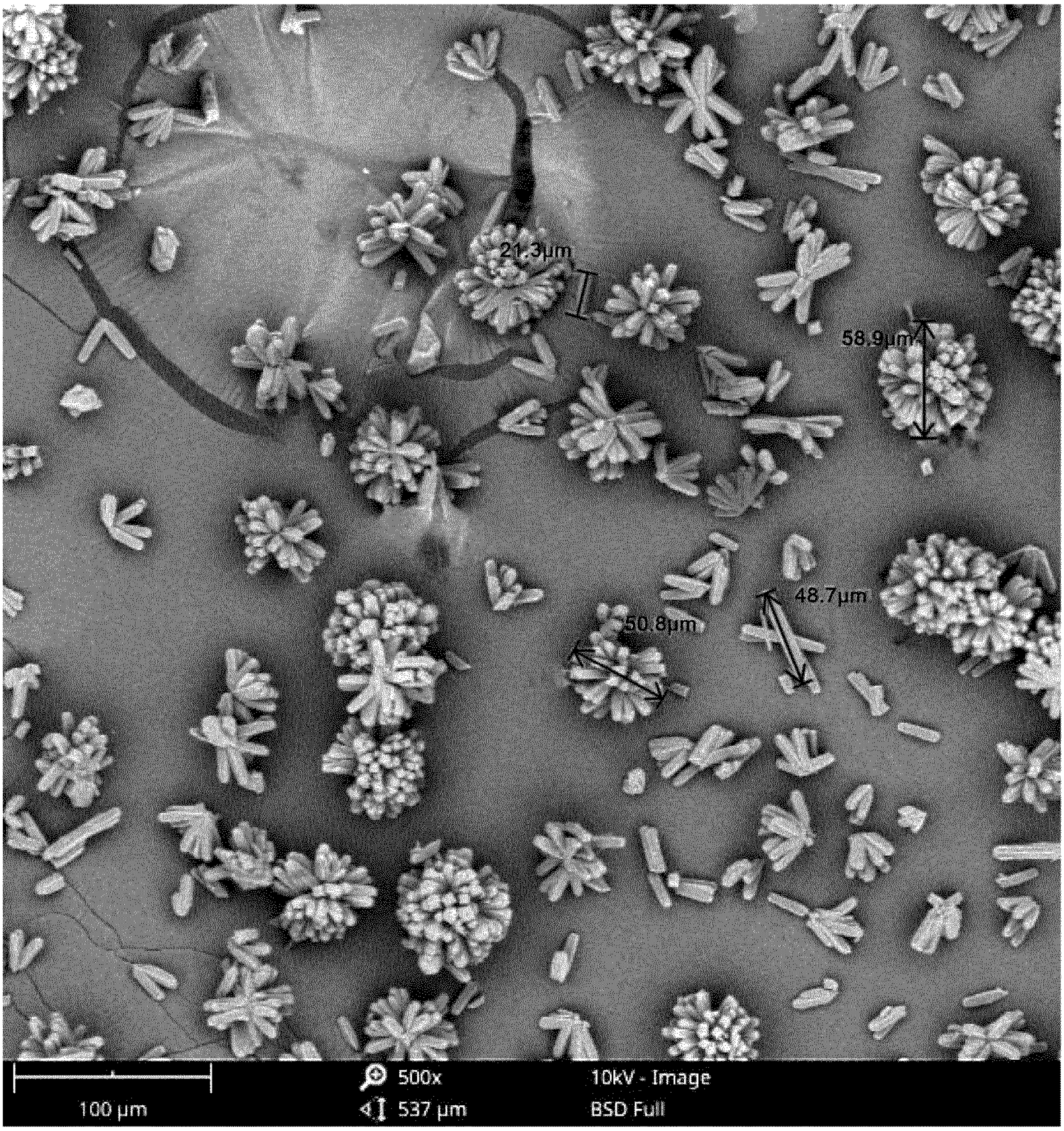
21.3μm
58.9μm
48.7μm
50.8μm
100 μm
500x
537 μm
10kV - Image
BSD Full

Fig.5

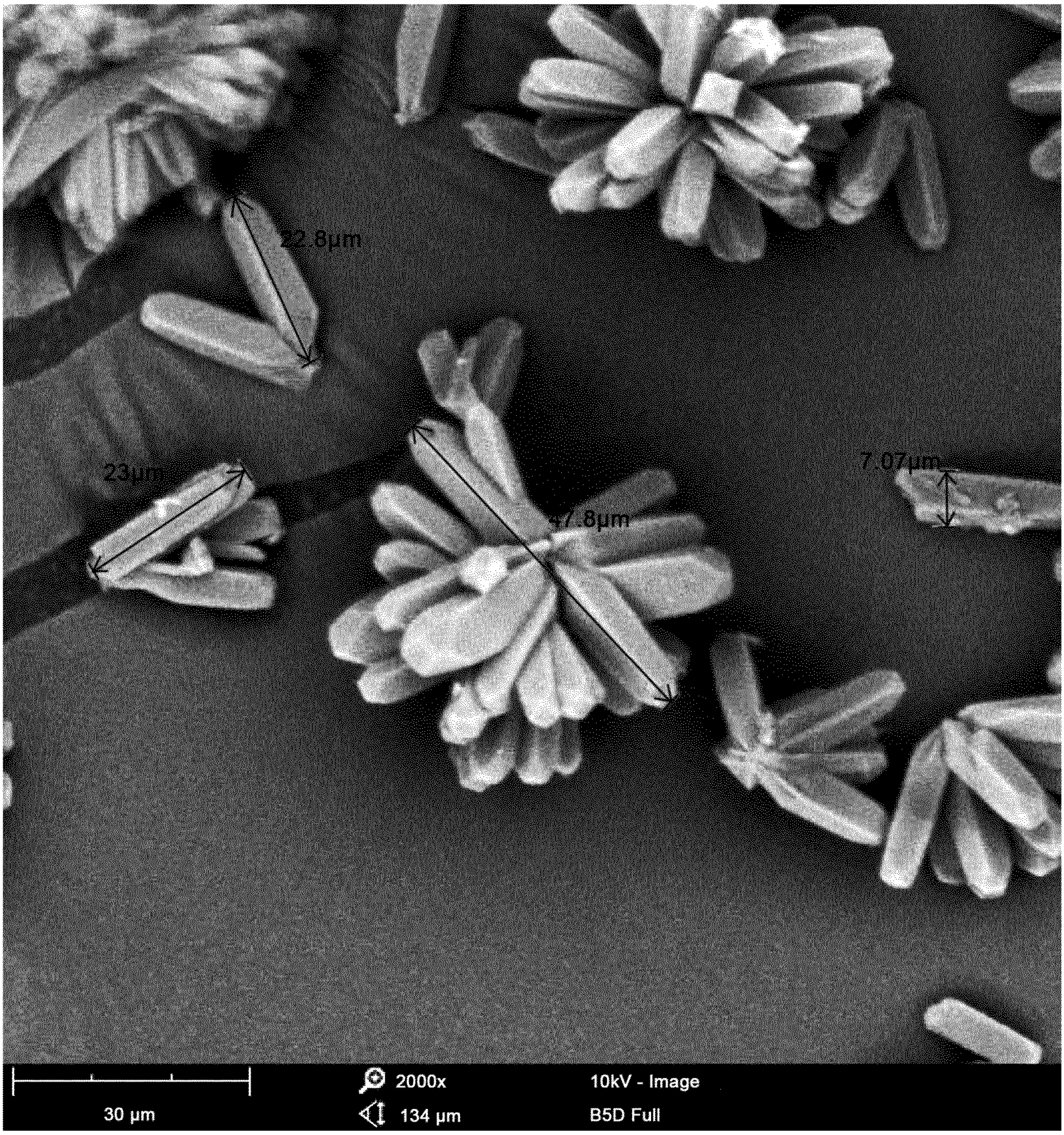


Fig.6

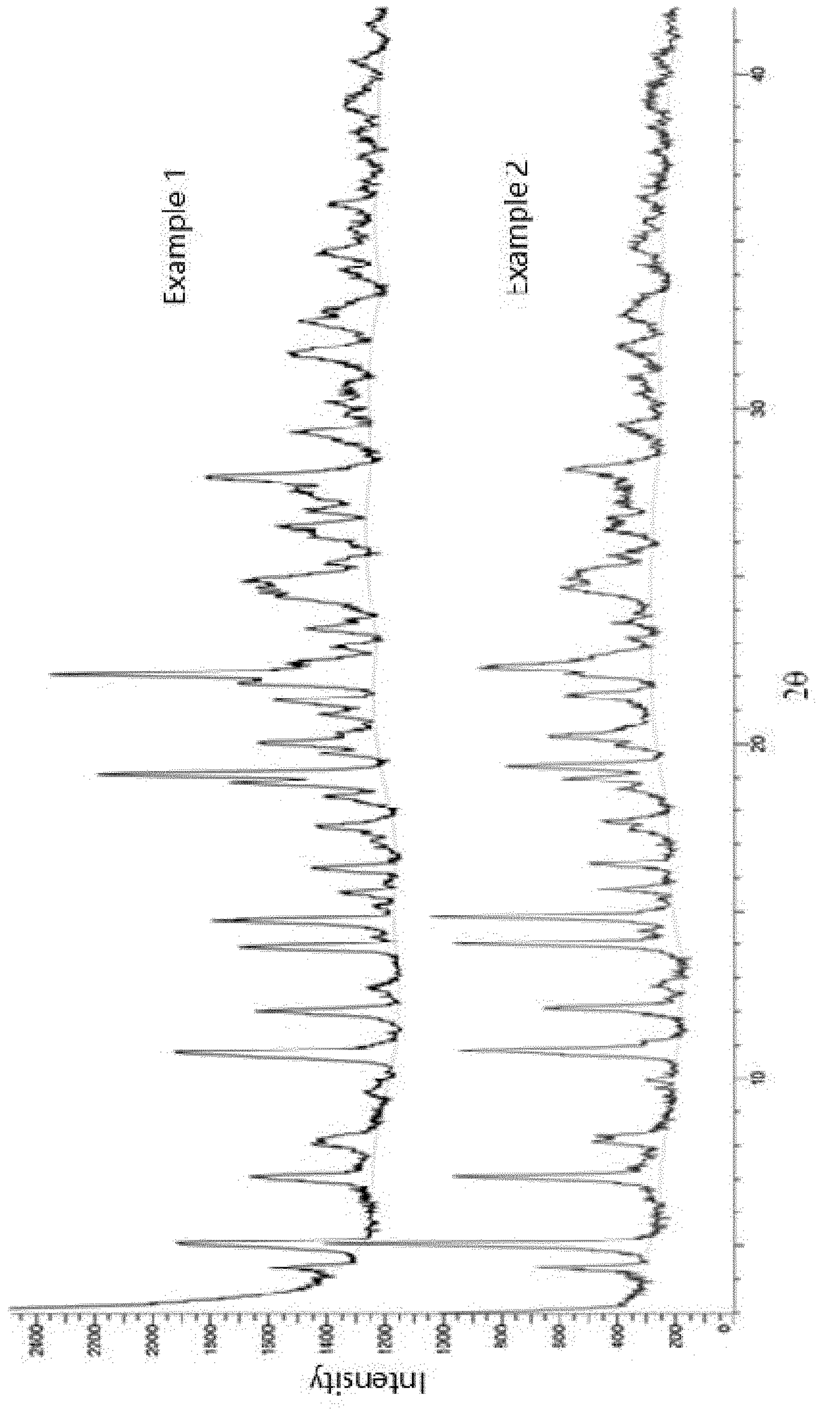


Fig.7

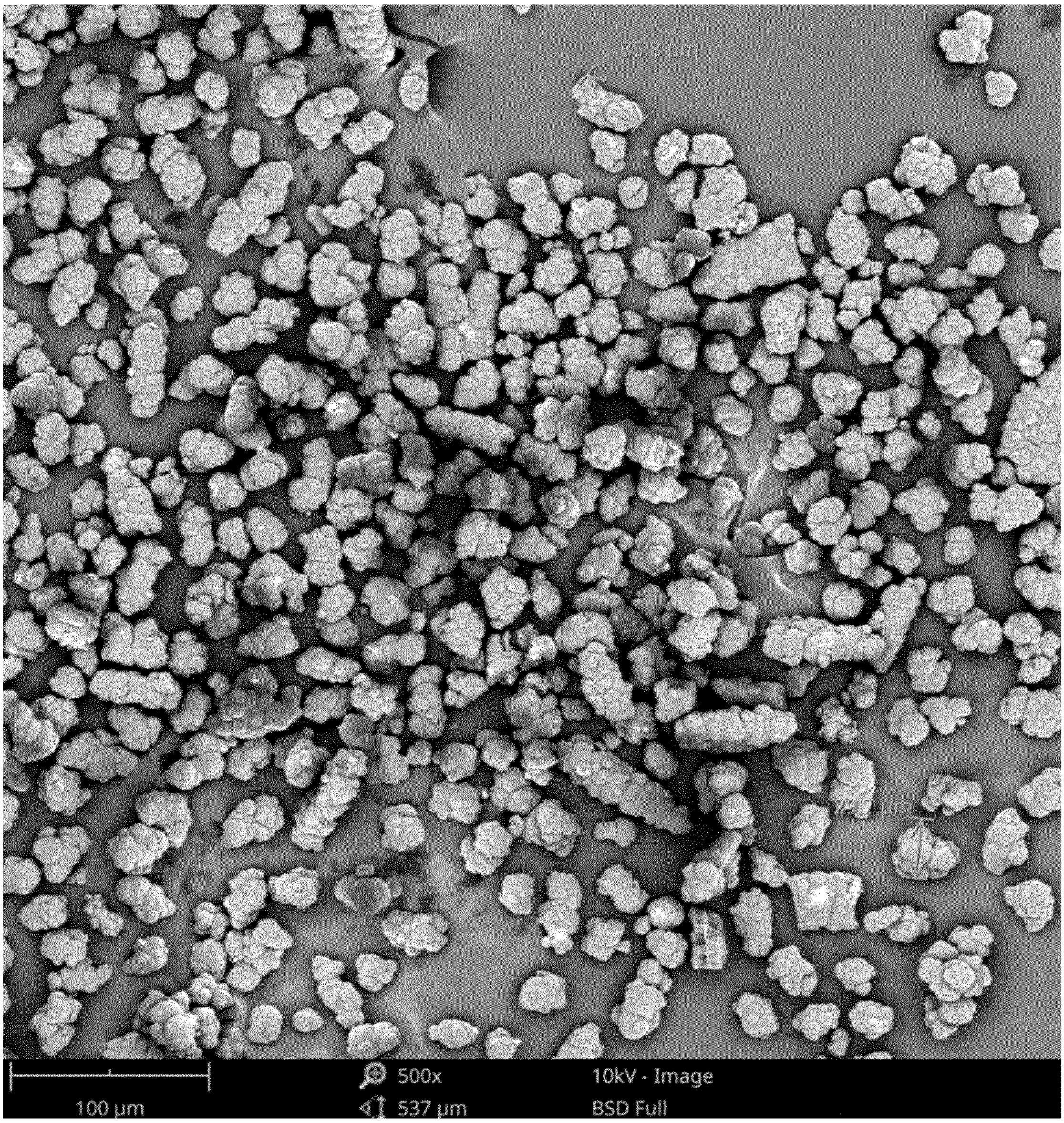


Fig.8

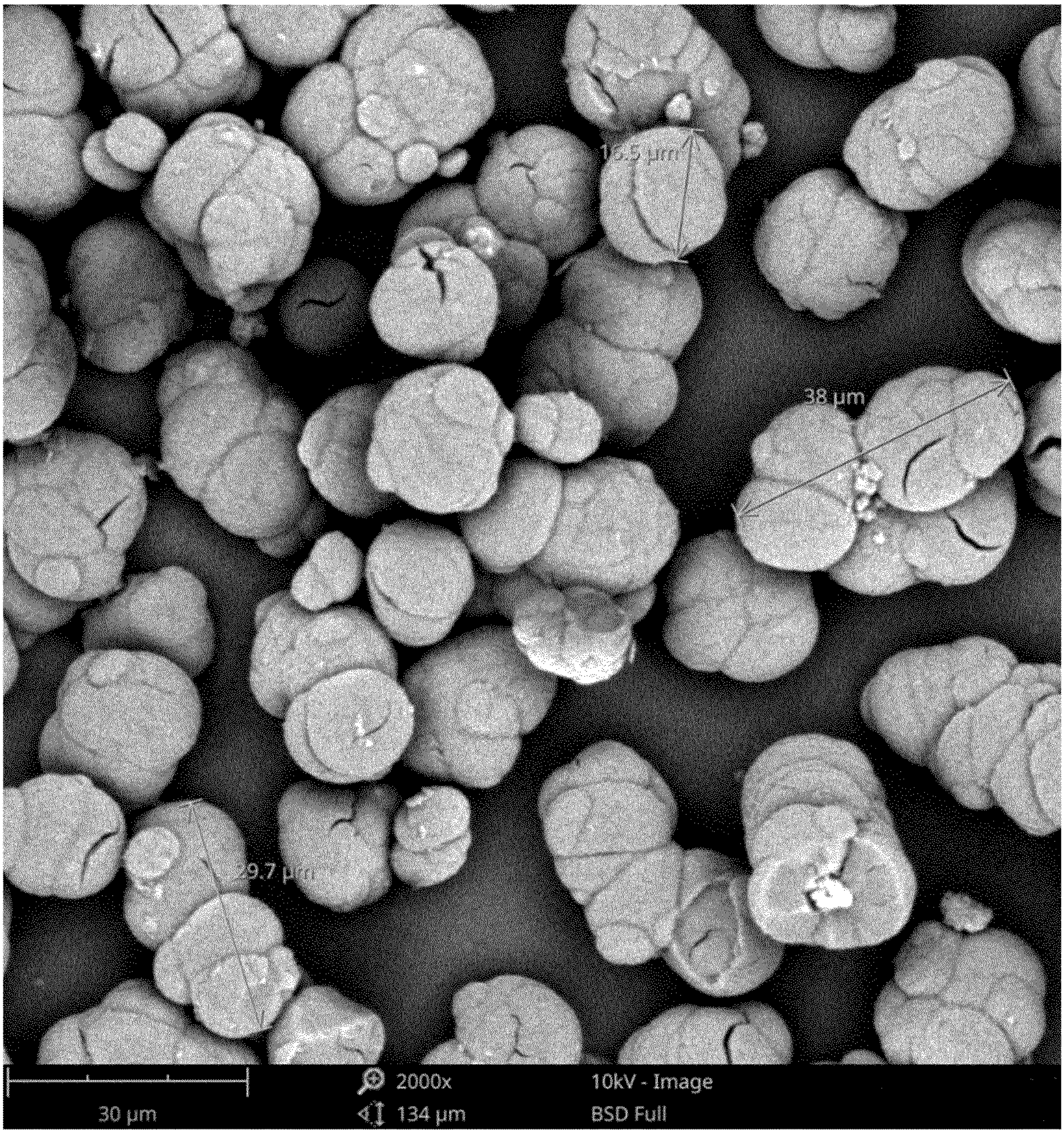


Fig.9

**INTERNATIONAL SEARCH REPORT**

International application No.
**PCT/CN2021/134856**

**A. CLASSIFICATION OF SUBJECT MATTER**

C07D 477/20(2006.01)i; C07D 477/02(2006.01)i

According to International Patent Classification (IPC) or to both national classification and IPC

**B. FIELDS SEARCHED**

Minimum documentation searched (classification system followed by classification symbols)

C07D477/-

Documentation searched other than minimum documentation to the extent that such documents are included in the fields searched

Electronic data base consulted during the international search (name of data base and, where practicable, search terms used)

WPABSC; CNTXT; ENTXT; CNKI: 凯莱英, 厄他培南钠, 晶体, 晶型, 碳青霉烯, 晶习, 棒状, 甲醇, 正丙醇, Ertapenem sodium, crystal, rodlike, crystal habit, carbapenem, methyl alcohol, n-propanol; REGISTRY(STN), CAPLUS(STN): perform structural formula search based on formula I

**C. DOCUMENTS CONSIDERED TO BE RELEVANT**

| Category* | Citation of document, with indication, where appropriate, of the relevant passages | Relevant to claim No. |
|---|---|---|
| PX | CN 113416193 A (ASYMCHEM LABORATORIES (TIANJIN) CO., LTD. et al.) 21 September 2021 (2021-09-21)<br>claims 1-12 | 1-12 |
| X | CN 102731502 A (CSPC ZHONGQI PHARMACEUTICAL TECHNOLOGY (SHIJIAZHUANG) CO., LTD.) 17 October 2012 (2012-10-17)<br>description, paragraphs 0076-0077 | 1-12 |
| X | CN 102363617 A (SHANGHAI CDYMAX PHARMACEUTICALS CO., LTD. et al.) 29 February 2012 (2012-02-29)<br>description, figure 1, embodiment 2, and table 2 | 1-12 |
| X | CN 102558182 A (CSPC ZHONGQI PHARMACEUTICAL TECHNOLOGY (SHIJIAZHUANG) CO., LTD.) 11 July 2012 (2012-07-11)<br>description, embodiment 1, and table 6 | 1-12 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **17 January 2022** | **26 January 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2021/134856**

| Patent document cited in search report | Publication date (day/month/year) | Patent family member(s) | Publication date (day/month/year) |
|---|---|---|---|
| CN 113416193 A | 21 September 2021 | None | |
| CN 102731502 A | 17 October 2012 | WO 2012139414 A1 | 18 October 2012 |
| CN 102363617 A | 29 February 2012 | WO 2013067878 A1 | 16 May 2013 |
| | | CN 102363617 B | 18 September 2013 |
| CN 102558182 A | 11 July 2012 | ES 2746045 T3 | 04 March 2020 |
| | | EP 2660242 A1 | 06 November 2013 |
| | | EP 2660242 A4 | 02 July 2014 |
| | | EP 2660242 B1 | 26 June 2019 |
| | | IN 1266MUMNP2013 A | 15 August 2014 |
| | | JP 2014501265 A | 20 January 2014 |
| | | CN 102558182 B | 11 February 2015 |
| | | US 2013281427 A1 | 24 October 2013 |
| | | US 9012628 B2 | 21 April 2015 |
| | | JP 2017025068 A | 02 February 2017 |
| | | JP 6321735 B2 | 09 May 2018 |
| | | RU 2013135276 A | 10 February 2015 |
| | | RU 2583052 C2 | 10 May 2016 |
| | | WO 2012089058 A1 | 05 July 2012 |
| | | KR 20140029367 A | 10 March 2014 |
| | | KR 101929960 B1 | 17 December 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03026572 A, Merck
- WO 03027067 A
- WO 2009150630 A
- WO 2013067878 A
- CN 1752090 A
- CN 102363617

### Non-patent literature cited in the description

- *j.org.Chem,* 2005, vol. 70, 7478-7487
- *CHEMICAL ABSTRACTS,* 153773-82-1